# EUROPEAN PATENT APPLICATION

(11) **EP 4 269 605 A1**
(43) Date of publication of application: **01.11.2023**
(21) Application number: 21915335.0
(22) Date of filing: 28.12.2021
(51) Int. Cl.: C12Q 1/56, G01N 33/48, G01N 33/86

(54) **BLOOD COAGULATION INSPECTION METHOD**

(30) Priority: 28.12.2020 JP 2020219441
(71) Applicant: Fujimori Kogyo Co., Ltd., Tokyo 112-0002 (JP)
(72) Inventor: HOSOKAWA, Kazuya, Tokyo 112-0002 (JP); NAGASATO, Tomoka, Tokyo 112-0002 (JP); KANEKO, Hisayo, Tokyo 112-0002 (JP); OYAMADA, Chiaki, Tokyo 112-0002 (JP); WADA, Tomoko, Tokyo 112-0002 (JP)
(74) Representative: Jones, Nicholas Andrew
(86) International application number: PCT/JP2021/048980
(87) International publication number: WO 2022/145477

(57) **Abstract**

A method of analyzing blood coagulation in vitro, comprising analyzing blood coagulation ability using a blood sample in which an extrinsic blood coagulation activator, thrombomodulin and a heparin-like substance are added.

## Description

### [Technical Field]

The present invention relates to a blood coagulation analysis method.

### [Background Art]

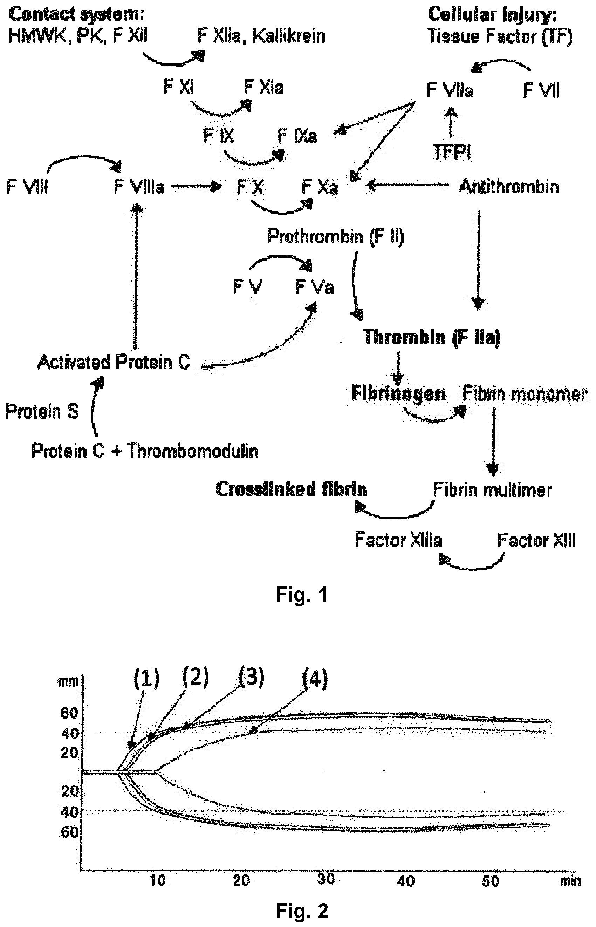

Thrombomodulin (TM) is a glycoprotein which has anticoagulation ability and is expressed in vascular endothelial cells, and when thrombin produced by activated blood coagulation binds to thrombomodulin, substrate specificity of thrombin changes, which thereby reduces the ability of thrombin to convert fibrinogen to fibrin and effectively activates protein C (PC), resulting in creating activated PC (APC). APC exhibits its anticoagulant effect by decomposing an activated blood coagulation fifth factor (FVa) and an activated blood coagulation eighth factor (FVIIIa). Moreover, protein S (PS) promotes an inactivation reaction of FVa and FVIIIa by APC as a coenzyme of APC (Figure 1).

A mutation of FVa is known, on the other hand, to cause disorders such as thrombosis, which is a resistant mutation to APC that prevents inactivation of FVa by APC, thereby impairing anticoagulant action by the TM/APC pathway. It is called an FV Leiden mutation and is known as a major thrombogenic predisposition in Caucasians.

In order to detect such disorders via the TM pathway in a blood analysis, a blood coagulation analysis can be performed by adding TM to a blood sample. It is reported about a blood coagulation analysis using TM that the amount of thrombin produced and the peak height of a thrombin concentration were inhibited in a thrombin production test. However, the thrombin production test requires the separation of plasma. With regard to a blood coagulation analysis using whole blood, the effect of shortening an activated clotting time (ACT) is not enough even with a high concentration of TM, and a high concentration of TM is also required for prolongation of a clotting time in the analysis of a thromboelastograph (Non-Patent Literature 1 and 2).

### [Citation List]

### [Non-Patent Literature]

[Non-Patent Literature 1]
   Pharmacological Differentiation of Thrombomodulin Alfa and Activated Protein C on Coagulation and Fibrinolysis In Vitrohttps://www.ncbi.nlm.nih.gov/pmc/articles/PMC6714727/
[Non-Patent Literature 2]

The use of thromboelastography (TEG) in massively bleeding patients at Haukeland University Hospital 2008-15 February 2019 Transfusion and Apheresis Science 58(1)

### [Summary of Invention]

### [Problem to be solved by invention]

An object of the present invention is to provide a method capable of efficiently evaluating the thrombomodulin pathway, in particular functions of protein C and protein S, in a blood coagulation test.

### [Solution to solve the problem]

The present inventors intensively studied in order to solve the above problem. As a result, the present inventors have found that blood coagulation ability reflecting functions of protein C and protein S can be easily evaluated by adding an extrinsic blood coagulation activator, thrombomodulin and a heparin-like substance to a blood sample to test the blood coagulation ability, and thus have completed the present invention.

One aspect of the present invention relates to a method of analyzing blood coagulation ability in vitro, comprising analyzing blood coagulation ability using a blood sample to which an extrinsic blood coagulation activator, thrombomodulin and a heparin-like substance are added.

The extrinsic blood coagulation activator may be a tissue factor or tissue thromboplastin.

The heparin-like substance may be one or more sulfated polysaccharides selected from the group consisting of an unfractionated heparin, a low molecular weight heparin, sodium fondaparinux, heparan sulfate and dextran sulfate.

The final concentration of thrombomodulin may be from 9 nM to 200 nM.

Thrombomodulin may be a soluble thrombomodulin.

In one aspect of the method of the present invention, functions of protein C and protein S can be evaluated by performing blood coagulation ability analysis with further adding a protein C inhibitor and comparing the analysis result with that obtained when the protein C inhibitor is not added.

In one aspect of the method of the present invention, if the blood sample is subjected to an anticoagulation treatment with citric acid, blood coagulation can be evaluated by adding calcium, an extrinsic blood coagulation activator, thrombomodulin and a heparin-like substance.

In one aspect of the method of the present invention, the blood coagulation ability can be evaluated by adding an extrinsic blood coagulation activator and a contact factor inhibitor.

In one aspect of the method of the present invention, a contact factor inhibitor may be either an FXII inhibitor or a kallikrein inhibitor.

In an aspect of the method of the present invention, the functions of protein C and protein S can be evaluated by comparing the analysis result with that obtained when thrombomodulin is not added.

In one aspect of the method, blood coagulation can be evaluated by setting a blood clotting time in the case of not adding thrombomodulin (i.e., blood to which a tissue factor or tissue thromboplastin and a heparin-like substance are added) to 60 seconds to 700 minutes and adding thrombomodulin thereto.

### [Advantageous Effects of Invention]

According to the method of the present invention, which is analysis of extrinsic blood coagulation, the addition of thrombomodulin and a heparin-like substance enhances an effect of thrombomodulin on prolongation of blood clotting time and thus the thrombomodulin function, more specifically, the functions of PC and PS can be evaluated. The addition of a heparin-like substance enables analysis of prolongation of a blood clotting time with a small amount of thrombomodulin, which is preferred.

### [Brief description of Drawings]

[Figure 1] A schematic view of a blood coagulation cascade.
[Figure 2] A view illustrating the results of Example 1.
[Figure 3] A view illustrating the results of Example 2.
[Figure 4] A view illustrating the results of Example 3.
[Figure 5] A view illustrating the results of Example 4.
[Figure 6] A view illustrating the results of Example 5.
[Figure 7] A view illustrating the results of Example 6.
[Figure 8] A view illustrating the results of Example 7.
[Figure 9] A view illustrating the results of Example 8.
[Figure 10] A view illustrating the results of Example 9
[Figure 11] A view illustrating the results of Example 10.

### [Description of Embodiments]

The method of analyzing blood coagulation of the present invention is characterized in that blood coagulation ability is evaluated by adding an extrinsic blood coagulation activator, thrombomodulin and a heparin-like substance to a blood sample.

Addition of the heparin-like substance together with thrombomodulin enables analysis of blood coagulation ability reflecting the functions of protein C and protein S.

A blood sample is preferably a whole blood sample or a platelet-rich plasma, and it may be a blood sample that is subjected to anticoagulation treatment with citric acid or the like. In such cases, the anticoagulant treatment can be released with calcium or the like to start a blood coagulation reaction.

In the method of the present invention, thrombomodulin is preferably added at a final concentration ranging from 9 nM to 200 nM.

Thrombomodulin being less than 9 nM may not clearly exert an anticoagulant effect even in the case of adding a tissue factor or a tissue thromboplastin and a heparin-like substance. Moreover, a concentration as high as 200 nM enables thrombomodulin to exhibit an anticoagulant ability even without the addition of the heparin-like substance, however, thrombomodulin is expensive, giving rise to a problem for diagnostic use.

A soluble thrombomodulin lacking a transmembrane region is preferably used as thrombomodulin. The soluble thrombomodulin is produced by being purified from blood or by genetic recombination technology, however, for example, Recomodulin (manufactured by Asahi Kasei Pharma Corporation) can be used as a recombinant soluble thrombomodulin.

Examples of the extrinsic blood coagulation activator include a tissue factor and a tissue thromboplastin.

The tissue factor and tissue thromboplastin may have variable specific activities, and thus, for example, tissue factor and/or tissue thromboplastin are preferably added to whole blood such that a blood clotting time is 50 to 600 seconds.

Analysis on blood coagulation in the presence of a low concentration of the tissue factor or tissue thromboplastin and heparan sulfate and thrombomodulin is preferable because the condition is close to the blood coagulation in vivo.

An embodiment of evaluating the blood coagulation ability by adding the extrinsic blood coagulation activators such as a low concentration tissue factor and tissue thromboplastin, and a contact factor inhibitor, as well as thrombomodulin and a heparin-like substance to a blood sample, is also preferred. Addition of the contact factor inhibitor makes it possible to evaluate physiological blood coagulation without the influence of a plastic container on blood coagulation.

In the case of analyzing a blood clotting time in a relatively short time (for example, 10 minutes or shorter), the influence of activation of a contact factor due to contact with a plastic container is minimal, whereas with a long analysis time of blood coagulation (for example, 30 minutes or longer) there may be an influence of shortened blood clotting time due to the activation of the contact factor.

Here, contact factor inhibitors include a blood coagulation 12 factor (FXII) inhibitor, a blood coagulation 11 factor (FXI) inhibitor, and a kallikrein inhibitor.

The FXII inhibitor and FXI inhibitor may be inhibitors against the activation of FXII and FXI or inhibitors against enzymatic activity of activated FXII (FXIIa) and activated FXI (FXIa).

The FXII inhibitor is not particularly limited as long as it is a substance with FXII inhibitory activity, and a corn trypsin inhibitor (CTI), a peptide inhibitor (J Med Chem. 2017 Feb 9; 60(3): 1151-1158), and the like can be used. The FXII inhibitor, for example, CTI, is preferably added at a final concentration of 10 to 100 µg/mL.

The kallikrein inhibitor is not particularly limited, and preferably a synthetic kallikrein inhibitor (PKSI-527 manufactured by FUJIFII,M Wako Pure Chemical Corporation) and aprotinin. A kallikrein inhibitor (PKSI-527) is preferably added at a final concentration of 0.1 µM to 100 µM.

Examples of the heparin-like substance include an unfractionated heparin, a low molecular weight heparin (for example, heparin with a mass-average molecular weight of 4,500 to 6,500), fondaparinux sodium (a pentasaccharide structure within heparin, promoting Xa inhibition of antithrombin: a product example: ARIXTRA) or sulfated polysaccharides such as heparan sulfate (for example, danaparoid sodium: a product example: ORGARAN), and dextran sulfate.

The heparan sulfate is present on vascular endothelial cells and is responsible for physiological anticoagulation in blood vessels. Therefore, the use of heparan sulfate as a heparin-like substance can reproduces physiological blood coagulation, which is more preferred. Heparan sulfate is preferably added at a final concentration of 0.4 to 15 ug/mL.

Moreover, in the case of evaluating blood samples from patients being treated with heparin, heparin-like substances such as sodium fondaparinux or dextran sulfate can be used.

Basic substances such as protamine and polybrene are used as neutralizers of the unfractionated heparin, but sodium fondaparinux is not neutralized by protamine and polybrene. Therefore, when fondaparinux sodium is used as a heparin-like substance, only the unfractionated heparin can be specifically neutralized by protamine and polybrene.

In addition, heparinase is used to decompose heparin and the low molecular weight heparin, but dextran sulfate is not susceptible for decomposition by heparinase. Therefore, when dextran sulfate is used as a heparin-like substance, only the unfractionated heparin and low molecular weight heparin can be specifically decomposed by heparinase.

Thus, in a case in which blood samples containing heparin and the low molecular weight heparin, such as blood of patients being treated with heparin, are inspected, the use of fondaparinux sodium or dextran sulfate as a heparin-like substance enables the heparin and low molecular weight heparin in the blood to be specifically neutralized or decomposed.

As a final concentration, for the unfractionated heparin, it is preferably added in a concentration of 0.01 to 1 U/mL, for the low molecular weight heparin, it is preferably added in a concentration of 0.01 to 2 U/mL, and for the fondaparinux sodium, it is preferably added in a concentration of 0.1 to 10 µg/mL.

Preferably, these heparin-like substances are added together with a tissue factor or tissue thromboplastin and a contact factor inhibitor so as to attain a blood clotting time of 60 to 700 seconds. The clotting time of 60 to 700 seconds renders suitable prolongation of the blood clotting time upon addition of thrombomodulin (9 to 200 nM), and allows for evaluation of anticoagulation ability by thrombomodulin.

A method of analyzing blood coagulation is not particularly limited, and is preferably by such apparatus that can evaluate a viscoelasticity of whole blood, including ROTEM (Rotational Thromboelastometry: Instrumentation Laboratory Co (IL)), TEG (Thromboelastography), SONOCLOT (Scientco Inc.)), and a variable angle type thromboelastography and graph analysis apparatus (blood coagulation inspection apparatus and blood coagulation inspection methods; WO2018/043420), and such apparatus that can analyze blood coagulation such as thrombin production in plasma in detail.

Whether abnormal coagulation is present in a sample can be checked by preliminarily adding each of an extrinsic blood coagulation activator, thrombomodulin and a heparin-like substance to healthy blood and blood of patients with abnormal coagulation and performing a blood coagulation test to calculate a clotting time and a coagulation waveform, and then observing whether the clotting time or waveform of a sample is close either of those of the aforementioned healthy blood or blood of patients.

The functions of protein C and protein S can also be evaluated by comparing the results of a blood coagulation test performed by addition of an extrinsic blood coagulation activator, thrombomodulin and a heparin-like substance to a blood sample, with those performed by having added no thrombomodulin (in the absence of thrombomodulin), i.e., those performed by addition of the extrinsic blood coagulation activator and the heparin-like substance to a blood sample.

For example, the functions of protein C and protein S can be evaluated by comparing a blood clotting time and a ROTEM/TEG waveform with and without thrombomodulin, and evaluating the extent of prolongation.

The difference corresponds to the effect of thrombomodulin, and no difference observed between the two results can conclude that a quantitative or functional impairment of protein C and/or protein S is likely to be present.

For example, FV Leiden that is an anomaly in which FVa is not inactivated by APC (activated protein C) due to a mutation in the gene of FV, can be a factor in thrombosis, but is not reflected in blood clotting time analysis in the absence of thrombomodulin, and therefore the difference between blood clotting times in the presence and absence of soluble thrombomodulin, becomes small.

Moreover, protein S (PS) is present in blood as both free PS and a complex with complement regulatory factor C4BP (PS-C4BP complex). The free PS acts as a cofactor for APC. When C4BP increases due to inflammation and the like, free PS decreases, and the anticoagulant ability of APC is reduced. In such conditions, the difference in blood clotting times in the presence and absence of soluble thrombomodulin, becomes small.

Thus, the functions of PC and PS can be analyzed by comparing blood coagulation in the presence and absence of thrombomodulin.

Incidentally, when blood coagulation is compared in the presence and absence thrombomodulin, the starting time of blood coagulation in the absence of thrombomodulin is preferably adjusted to be from 60 to 700 seconds. A clotting time in the absence of thrombomodulin within 60 seconds causes immediate autocoagulation by a large amount of thrombin produced in a short time, making PC and PS function analysis difficult because the blood coagulation reaction is completed before the anticoagulation effect via APC by addition of thrombomodulin is exhibited. A composition having a clotting time of 700 seconds or longer in the absence of thrombomodulin, on the other hand, makes PC and PS analysis difficult because addition of thrombomodulin completely inhibits blood coagulation by thrombomodulin.

In this case, adding more extrinsic activator shortens a clotting time, and adding more heparin-like substance delays a clotting time, thereby enabling the time to be adjusted to from 60 to 700 seconds by taking a balance therebetween.

Thrombomodulin has two types of anticoagulation ability: i.e., the anticoagulant activity by directly inhibiting fibrin production of thrombin, and FVIII and FV activation ability, and the anticoagulant activity by decomposition promotion of FVIIIa and FVa by converting protein C (PC) to activated protein C (APC). Therefore, the anticoagulant activity via PC/PS can be specifically analyzed by comparing blood coagulation in the presence of thrombomodulin with and without an inhibitor of APC.

The inhibitor of APC (or inhibitor of PC) includes a substance that inhibits PC activation or a substance that inhibits the enzymatic activity of APC, but it is not particularly limited, and, for example, a DNA aptamer of APC inhibition can be used. Examples of APC inhibitory aptamers include HS02-44G (Chemistry & Biology Volume 16, Issue 4, 24 April 2009, Pages 442-451), but is not particularly limited thereto.

The present invention also provides a blood coagulation analysis reagent containing the extrinsic blood coagulation activator, thrombomodulin and the heparin-like substance. The extrinsic blood coagulation activator is preferably a tissue factor or tissue thromboplastin. The heparin-like substance is preferably the unfractionated heparin, the low molecular weight heparin, fondaparinux sodium (ARXTRA), and heparan sulfate and dextran sulfate.

The blood coagulation analysis reagent may further contain contact factor inhibitors such as the FXII inhibitor and kallikrein inhibitor.

The blood coagulation analysis reagent may further contain a protein C inhibitor.

The preferred concentration (final concentration) of each component upon use is as described above and thus each component is diluted to a preferred final concentration upon use (when a blood sample is added).

Each component may be preliminarily mixed, however, it is preferably included as respective reagent components in a blood coagulation analysis reagent kit and be mixed in an appropriate concentration for analysis upon use. The blood coagulation analysis reagent may include instructions for use for blood coagulation analysis.

### [Examples]

The present invention will be described in more detail by referring to Examples below. However, the present invention is not limited to the aspects of the following Examples.

### Example 1

ROTEM (manufactured by IL) was used to analyze blood coagulation.

Blood was collected using a blood collection tube containing 3.2% sodium citrate manufactured by Terumo Corporation.

Blood coagulation was analyzed by having added 20 µL of Extern reagent (manufactured by IL) that was a 50-fold diluted extrinsic activator, 20 µL of a Startem reagent (IL) that was a calcium chloride reagent (final concentration 12 mM), and a soluble thrombomodulin (rTM; trade name: Recomodulin manufactured by Asahi Kasei Pharma Corporation) (final concentrations thereof were 0, 20 nM, 50 nM, and 200 nM, respectively) to 300 µL of whole blood.

The results are shown in Figure 2.
(1) 1/50 Extern + Startem
(2) 1/50 Extern + Startem + rTM 20 nM
(3) 1/50 Extern + Startem + rTM 50 nM
(4) 1/50 Extern + Startem + rTM 200 nM

The blood clotting times (CT) of blood with 0, 20, 50, and 200 nM of soluble thrombomodulin added were 275, 323, 342, and 570 seconds, respectively. Even at 50 nM of soluble thrombomodulin, the prolongation effect of the blood clotting time was limitative. 1.5 times or more prolongation requires the addition of 200 nM of soluble thrombomodulin.

### Example 2

ROTEM was used to analyze blood coagulation.

Blood was collected using a blood collection tube containing 3.2% sodium citrate manufactured by Terumo Corporation.

Blood coagulation was analyzed in the same manner as in Example 1 except that a 50-fold dilution of a rabbit brain-derived tissue thromboplastin (TP) reagent (final concentration 0.9 µg/mL) had been added to blood instead of the 50-fold diluted EXTEM reagent.

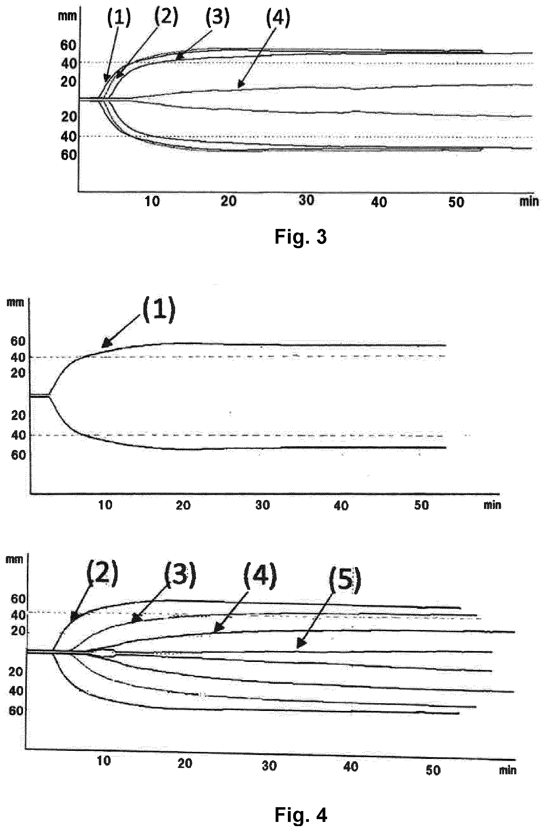

The results are shown in Figure 3.
(1) TP (0.9 µg/mL) + Startem
(2) TP (0.9 µg/mL) + Startem + rTM 20 nM
(3) TP(0.9µg/mL) + Startem + rTM 50 nM
(4) TP(0.9µg/mL) + Startem + rTM 200 nM

The blood clotting times (CT) of blood with 0, 20, 50, and 200 nM of soluble thrombomodulin were 157, 198, 243, and 428 seconds, respectively. Even in the case of having used the rabbit brain-derived thromboplastin reagent for extrinsic coagulation activation, the prolongation effect of 20 nM of soluble thrombomodulin on blood clotting times was limitative. The soluble thrombomodulin at 200 nM markedly prolonged blood coagulation.

### Example 3

ROTEM was used to analyze blood coagulation.

Blood was collected using a blood collection tube containing 3.2% sodium citrate manufactured by Terumo Corporation.

Blood coagulation waveforms were analyzed in the same experiment as in Example 2 except that a rabbit brain-derived tissue thromboplastin (TP: final concentration 0.9 µg/mL) and a soluble thrombomodulin (rTM: final concentration 20 nM) were added to blood, and heparan sulfate (manufactured by Iduron Ltd., average molecular weight 5500) had been added at final concentrations of 1 µg/mL, 1.5 µg/mL and 2 µg/mL, respectively.

The results are shown in Figure 4.
(1) TP (0.9 µg/mL) + Startem
(2) TP (0.9 µg/mL) + Startem + rTM 20 nM
(3) TP (0.9 µg/mL) + Startem + rTM 20 nM + heparan sulfate 1 µg/mL
(4) TP (0.9 µg/mL) + Startem + rTM 20nM + heparan sulfate 1.5 µg/mL
(5) TP (0.9 µg/mL) + Startem + rTM 20 nM + heparan sulfate 2 µg/mL

The clotting time (CT) of blood without the soluble thrombomodulin was 157 seconds, whereas that of blood with 20 nM of soluble thrombomodulin was 198 seconds. Furthermore, the CT of blood with 20 nM of soluble thrombomodulin and 1 µg/mL of heparan sulfate was 319 seconds, indicating that the effect of the soluble thrombomodulin was significantly enhanced in the presence of heparan sulfate.

Furthermore, in the case of having added 2 µg/mL of heparan sulfate, a small amount of soluble thrombomodulin significantly delayed the clotting time as well as inhibited the intensity of clot.

These results indicate that the addition of the extrinsic blood coagulation activator and the heparin-like substance greatly enhances the anticoagulation ability of thrombomodulin.

### Example 4

Blood coagulation was analyzed in the same experiment as in Example 3 by having added heparan sulfate (final concentration 1 µg/mL) together with an FXII inhibitor (CTI; final concentration 20 µg/mL) and a kallikrein inhibitor (PKSI-527; final concentration 40 µM) as contact factor inhibitors to a rabbit brain-derived tissue thromboplastin (TP; final concentration 0.9 µg/mL) in the presence and absence of a soluble thrombomodulin (rTM; final concentration 20 nM).

20 µL of calcium chloride solution (Ca; final concentration 12 mM) was used instead of a Startem reagent.

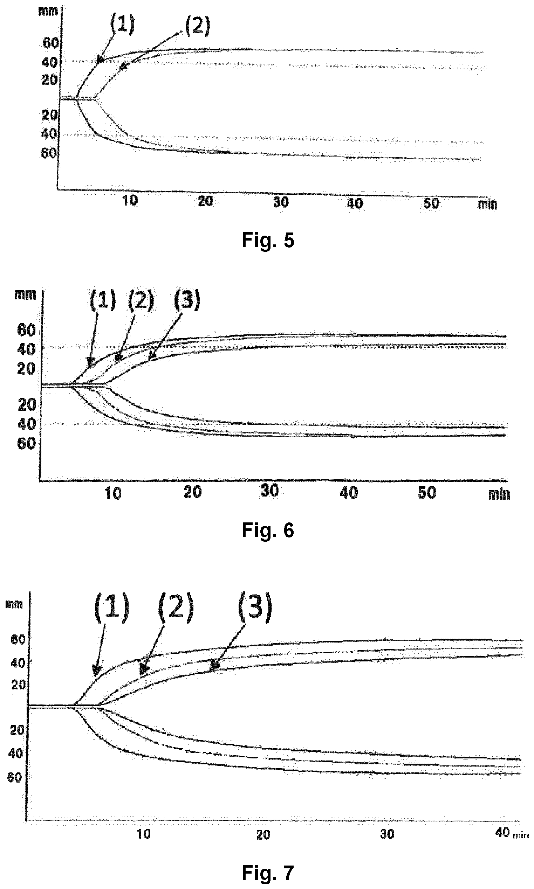

Results are shown in Figure 5.
(1) TP (0.9 µg/mL) + Ca + heparan sulfate + CTI + kallikrein inhibitor
(2) TP (0.9 µg/mL) + Ca + heparan sulfate + CTI + kallikrein inhibitor + rTM20nM

The clotting time (CT) of blood with heparan sulfate (1 µg/mL), contact factor inhibitors (the FXII inhibitor (CTI; 20 µg/mL), and the kallikrein inhibitor (PKSI-527; 40 µM)) was 162 seconds, whereas the CT upon further addition of 20 nM of soluble thrombomodulin was 290 seconds.

The addition of the soluble thrombomodulin at a low concentration (20 nM) to blood with the diluted rabbit brain-derived tissue thromboplastin, heparan sulfate and contact factor inhibitors markedly prolonged the clotting time.

### Example 5

Blood coagulation waveforms were analyzed in the same experiment as in Example 3 by having added an unfractionated heparin (Mochida Pharmaceutical Co., Ltd.; final concentration 0.1 U/mL) instead of heparan sulfate, with a soluble thrombomodulin (rTM) at final concentrations of 0 nM, 20 nM, and 30 nM, respectively.

The results are shown in Figure 6.
(1) TP (0.9 µg/mL) + Startem + unfractionated heparin 0.1 U/mL
(2) TP (0.9 µg/mL) + Startem + unfractionated heparin 0.1 U/mL + rTM 20 nM
(3) TP (0.9 µg/mL) + Startem + unfractionated heparin 0.1U/mL + rTM 30 nM

### Example 6

Blood coagulation was analyzed in the same experiment as in Example 5 by having used a low molecular weight heparin (product name FRAGMIN, manufactured by Kissei Pharmaceutical Co., Ltd. (sales) and Pfizer Inc. (manufacturer and distributor), final concentration 0.2 U/mL) instead of an unfractionated heparin.

The results are shown in Figure 7.
(1) TP (0.9 µg/mL) + Startem + FRAGMIN 0.2 U/mL
(2) TP (0.9 µg/mL) + Startem + P FRAGMIN 0.2 U/mL + rTM 20 nM
(3) TP (0.9 µg/mL) + Startem + P FRAGMIN 0.2U/mL + rTM 30 nM

### Example 7

Blood coagulation was analyzed in the same experiment as in Example 5 by having added ORGARAN (manufactured by Kyowa Critical Care Co., Ltd.; final concentration 0.2 U/mL) instead of an unfractionated heparin.

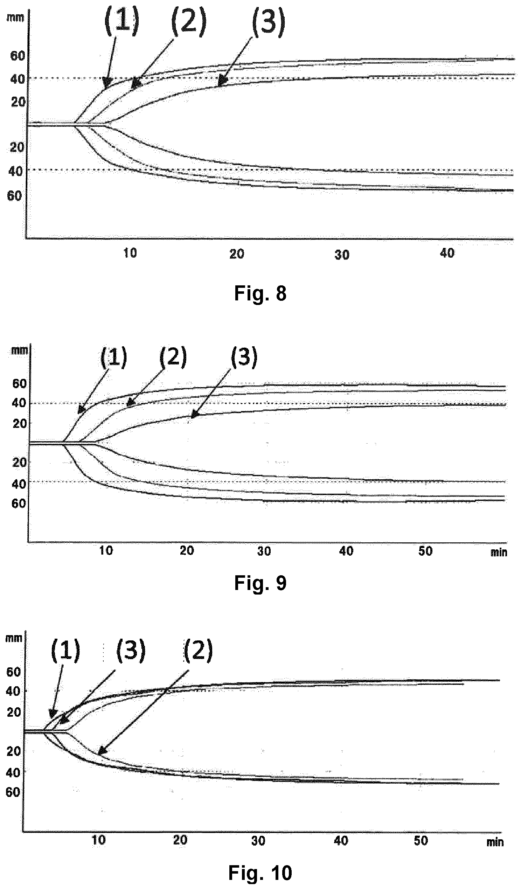

The results are shown in Figure 8.
(1) TP (0.9 µg/mL) + Startem + ORGARAN 0.2 U/mL
(2) TP (0.9 µg/mL) + Startem + ORGARAN 0.2 U/mL + rTM 20 nM
(3) TP (0.9 µg/mL) + Startem + ORGARAN 0.2 U/mL + rTM 30 nM

### Example 8

Blood coagulation was analyzed in the same experiment as in Example 5 by having added ARIXTRA (Aspen Japan Co., Ltd.; 0.5 µg/mL) instead of an unfractionated heparin. Results are shown in Figure 9.
(1) TP (0.9 µg/mL) + Startem + ARIXTRA 0.5 µg/mL
(2) TP (0.9 µg/mL) + Startem + ARIXTRA 0.5 µg/mL + rTM 20 nM
(3) TP (0.9 µg/mL) + Startem + ARIXTRA 0.5 µg/mL + rTM 30 nM

Blood coagulation was markedly prolonged by the addition of 20 nM or 30 nM of soluble thrombomodulin even in the case of having added the unfractionated heparin, low molecular weight heparin or pentasaccharide (ARIXTRA) instead of heparan sulfate in the experiments of Examples 4 to 8.

### Example 9

ROTEM was used to analyze blood coagulation.

Blood was collected using a blood collection tube containing 3.2% sodium citrate manufactured by Terumo Corporation.

Blood coagulation was analyzed by having added a rabbit brain-derived tissue thromboplastin reagent (TP; final concentration 0.9 µg/mL), a Startem reagent (calcium chloride: final concentration 12 mM), and heparan sulfate (1 µg/mL).

Blood coagulation was analyzed as a comparison by having added a soluble thrombomodulin (rTM; final concentration 20 nM) or a soluble thrombomodulin (rTM; final concentration 20 nM) and an APC inhibitory aptamer (HS02-44G; final concentration 1 µM; Chemistry & Biology Volume 16, Issue 4, 24 April 2009, Pages 442-451) to a rabbit brain-derived tissue thromboplastin reagent (TP; 0.9 µg/mL) and a Startem reagent (calcium chloride: final concentration 12 mM) and heparan sulfate (final concentration 1 µg/mL).

The results are shown in Figure 10.
(1) TP (0.9 µg/mL) + Startem + heparan sulfate 1 µg/mL
(2) TP (0.9 µg/mL) + Startem + heparan sulfate 1 µg/mL + rTM 20 nM
(3) TP (0.9 µg/mL) + Startem + heparan sulfate 1 µg/mL + rTM 20 nM + APC inhibitory aptamer 1 µM

The clotting time (CT) was prolonged from 154 to 319 seconds by having further added the soluble thrombomodulin (20 nM) to blood to which the tissue thromboplastin and heparan sulfate had been added.

The CT was also 208 seconds when the soluble thrombomodulin (20 nM) and the APC inhibitory aptamer had been further added to blood to which the tissue thromboplastin and heparan sulfate had been added.

From these results, the delay in CT from 154 to 208 seconds is considered to be due to the antithrombin action of the soluble thrombomodulin. The shortening from 319 to 208 seconds is considered to be due to the anticoagulant action via activated protein C. Therefore, comparing the clotting time with and without the APC inhibitory aptamer enables evaluation of the functions of PC/PS.

### Example 10

ROTEM was used to analyze blood coagulation.

Blood was collected using a blood collection tube containing 3.2% sodium citrate manufactured by Terumo Corporation.

Blood coagulation was analyzed by having added a 100-fold diluted rabbit brain-derived tissue thromboplastin reagent (TP; final concentration 0.45 µg/mL), a Startem reagent (calcium chloride; final concentration 12 mM), and heparan sulfate (final concentration 1 µg/mL).

Blood coagulation was analyzed as a comparison by having added a soluble thrombomodulin (rTM; final concentration 100 nM) or a soluble thrombomodulin (rTM; final concentration 100 nM) and an APC inhibitory aptamer (HS02-44G; final concentration 1 µM) to a rabbit brain-derived tissue thromboplastin reagent (0.9 µg/mL) and a Startem reagent (calcium chloride; final concentration 12 mM) and heparan sulfate (final concentration 1 µg/mL).

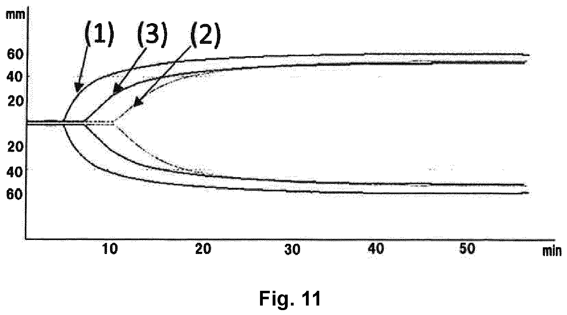

The results are shown in Figure 11.
(1) TP (0.45 µg/mL) + Startem + heparan sulfate 1 µg/mL
(2) TP (0.45 µg/mL) + Startem + heparan sulfate 1 µg/mL + rTM 100 nM
(3) TP (0.45 µg/mL) + Startem + heparan sulfate 1 µg/mL + rTM 100 nM + APC inhibitory aptamer 1 µM

The clotting time (CT) was prolonged from 251 to 593 seconds by having added the soluble thrombomodulin (100 nM) to blood to which the tissue thromboplastin and heparan sulfate had been added, which resulted in 2.36 times prolongation, and is considered to be due to both the "anticoagulant effect by antithrombin" and "anticoagulant effect via APC production" by thrombomodulin.

Moreover, the CT was 387 when the soluble thrombomodulin (100 nM) and the APC inhibitory aptamer further had been added to blood with tissue thromboplastin and heparan sulfate added, which specifically reflects the anticoagulant effect via APC.

In the presence of heparan sulfate, detailed analysis of blood coagulation can be performed by comparing each blood coagulation with thrombomodulin in the presence or absence of APC.

### Example 11

ROTEM was used to analyze blood coagulation.

Blood was collected using a blood collection tube containing 3.2% sodium citrate manufactured by Terumo Corporation.

Blood coagulation was analyzed in the case of having added a rabbit brain-derived tissue thromboplastin reagent (TP; final concentration 16.6 µg/mL), calcium chloride (Ca; final concentration 12 mM), heparan sulfate (final concentration 0, 1, 3, 10,15, 20, and 30 µg/mL), CTI (final concentration 20 µg/mL), and PKSI-527 (final concentration 40 µM) with and without a soluble thrombomodulin (rTM; final concentration 50 nM) further having been added.

The CT (clotting time) and CFT values (clot formation times) are shown in Table 1.

The prolongation effect of 50 nM of soluble thrombomodulin on the clotting time was not observed at 3 µg/mL or less of heparan sulfate, whereas the clotting time was significantly prolonged (1.5 times or more) by the addition of the soluble thrombomodulin at 10 and 15 µg/mL of heparin sulfate. When 20 or 30 µg/mL of heparan sulfate had been added, on the other hand, the CT value was not obtained by the addition of the thrombomodulin, and the blood clotting time was 1 hour or longer.

**[Table 1]**

| TP 16.6 µg/mL + Ca 12mM + CTI 20 µg/mL + PKSI-527 40µM+Heparan sulfate0~30 µg/mL±rTM 50nM | | | | | |
|---|---|---|---|---|---|
| | | no addition of rTM | | rTM 50 µM | |
| | | CT (sec) | CFT (sec) | CT (sec) | CFT (sec) |
| Heparan sulfate | 0 µg/mL | 50 | 92 | 46 | 96 |
| Heparan sulfate | 1 µg/mL | 58 | 93 | 55 | 72 |
| Heparan sulfate | 3 µg/mL | 62 | 93 | 62 | 73 |
| Heparan sulfate: | 10 µg/mL | 94 | 55 | 143 | 95 |
| Heparan sulfate | 15 µg/mL | 120 | 66 | 255 | 255 |
| Heparan sulfate | 20 µg/mL | 144 | 61 | No CT value | No CFT value |
| Heparan sulfate | 30 µg/mL | 193 | 69 | No CT value | No CFT value |

### Example 12

ROTEM was used to analyze blood coagulation.

Blood was collected using a blood collection tube containing 3.2% sodium citrate manufactured by Terumo Corporation.

Blood coagulation was analyzed in the case of having added a rabbit brain-derived tissue thromboplastin reagent (TP; final concentration 16.6 µg/mL), calcium chloride (Ca; final concentration 12 mM), heparan sulfate (final concentration 0,5 µg/mL), CTI (final concentration 20 µg/mL), and PKSI-527 (final concentration 40 µM), without and with soluble thrombomodulin (rTM; final concentration 100 nM) further having been added.

The CTs (clotting times) and CFT values (clot formation times) are shown in Table 2.

The clotting times were confirmed to be prolonged 1.5 times or more by the addition of the soluble thrombomodulin at 5 µg/mL of heparan sulfate.

**[Table 2]**

| TP 16.6 µg/mL+Ca 12mM + CTI 20 µg/mL + PKSI-527 40µM + Heparan sulfate 0∼5 µg/mL±rTM 100nM | | | | | |
|---|---|---|---|---|---|
| | | no addition of rTM | | rTM 100 µM | |
| | | CT (sec) | CFT (sec) | CT (sec) | CFT (sec) |
| Heparan sulfate | 0 µg/mL | 50 | 92 | 49 | 76 |
| Heparan sulfate | 5 µg/mL | 65 | 76 | 96 | 69 |

### Example 13

ROTEM was used to analyze blood coagulation.

Blood was collected using a blood collection tube containing 3.2% sodium citrate manufactured by Terumo Corporation.

Blood coagulation was analyzed in the case of having added a rabbit brain-derived tissue thromboplastin reagent (TP; final concentration 0.033 µg/mL), calcium chloride (Ca; final concentration 12 mM), heparan sulfate (final concentration 0, 0.5 µg/mL), CTI (final concentration 20 µg/mL) and PKSI-527 (final concentration 40 µM) without and with soluble thrombomodulin (rTM; final concentration 9 nM) further having been added.

The CTs (clotting times) and CFT values (clot formation times) are shown below.

For the case of having added the soluble thrombomodulin at a concentration of 9 nM, the clotting times were prolonged only to a limited extent where no heparan sulfate had been added, however, the clotting times were prolonged 1.5 times or more by having added 0.5 µg/mL of heparan sulfate.

**[Table 3]**

| TP 0.033 pg/mL+Ca 12mM+CTI 20 µg/mL+PKSI-527 40µM + Heparan sulfate 0∼0.5 µg/mL±rTM 9nM | | | | | |
|---|---|---|---|---|---|
| | | no addition of rTM | | rTM 9 nM | |
| | | CT (sec) | CFT (sec) | CT (sec) | CFT (sec) |
| Heparan sulfate | 0.0 µg/mL | 594 | 162 | 787 | 204 |
| Heparan sulfate | 0.5 µg/mL | 837 | 248 | 1355 | 1484 |

### Example 14

ROTEM was used to analyze blood coagulation.

Blood was collected using a blood collection tube containing 3.2% sodium citrate manufactured by Terumo Corporation.

Blood coagulation was analyzed in the case of having added a rabbit brain-derived tissue thromboplastin reagent (TP; final concentration 0.033 µg/mL), calcium chloride (Ca; final concentration 12 mM), heparan sulfate (final concentration 0, 0.1, 0.2, 0.3, 0.4, and 0.5 µg/mL), CTI (final concentration 20 µg/mL) and PKSI-527 (final concentration 40 µM) without and with a soluble thrombomodulin (rTM; final concentration 10 nM) further having been added.

The CTs (clotting times) and CFT values (clot formation times) are shown in Table 4.

When 10 nM of soluble thrombomodulin had been added, the prolongation of clotting time was limitative in the case of heparan sulfate being 0.3 µg/mL or less, however, the addition of heparan sulfate in a concentration of 0.4 µg/mL or higher prolonged the clotting time by more than 1.5 times.

**[Table 4]**

| TP 0.033 µg/mL + Ca 12mM + CTI 20 µg/ mL + PKSI-527 40µM + Heparan sulfate 0∼0.5 µg/mL ± rTM 10nM | | | | | |
|---|---|---|---|---|---|
| | | no addition of rTM | | rTM 10 nM | |
| | | CT (sec) | CFT (sec) | CT (sec) | CFT (sec) |
| Heparan sulfate | 0.0 µg/mL | 594 | 162 | 813 | 304 |
| Heparan sulfate | 0.1 µg/mL | 572 | 182 | 797 | 240 |
| Heparan sulfate | 0.2 µg/mL | 686 | 224 | 829 | 215 |
| Heparan sulfate | 0.3 µg/mL | 688 | 171 | 802 | 290 |
| Heparan sulfate | 0.4 µg/mL | 762 | 206 | 1360 | 733 |
| Heparan sulfate | 0.5 µg/mL | 837 | 248 | 1266 | 660 |

### Example 15

ROTEM was used to analyze blood coagulation.

Blood was collected using a blood collection tube containing 3.2% sodium citrate manufactured by Terumo Corporation.

Blood coagulation was analyzed in the case of having added a rabbit brain-derived tissue thromboplastin reagent (TP; final concentration 0.033 µg/mL), calcium chloride (Ca; final concentration 12 mM), heparan sulfate (final concentration 0, 0.1, 0.2, 0.3, 0.4, and 0.5 µg/mL), CTI (final concentration 20 µg/mL), and PKSI-517 (final concentration 40 µM) with and without a soluble thrombomodulin (rTM; final concentration 5 nM) further having been added.

The CTs (clotting times) and CFT values (clot formation times) are shown in Table 5.

The clotting times were not prolonged by 1.5 times or more when 5 nM of soluble thrombomodulin had been added, even in the case of the addition of heparan sulfate.

**[Table 5]**

| TP 0.033 µg/mL + Ca 12mM + CTI 20 µg/mL+PKSI-527 40µM +Heparan sulfate0~0.5 µg/mL±rTM 5 nM | | | | | |
|---|---|---|---|---|---|
| | | no addition of rTM | | rTM 5 nM | |
| | | CT (sec) | CFT (sec) | CT (sec) | CFT (sec) |
| Heparan sulfate | 0.0 µg/mL | 594 | 162 | 753 | 195 |
| Heparan sulfate | 0.1 µg/mL | 572 | 182 | 711 | 230 |
| Heparan sulfate | 0.2 µg/mL | 686 | 224 | 749 | 209 |
| Heparan sulfate | 0.3 µg/mL | 688 | 171 | 825 | 251 |
| Heparan sulfate | 0.4 µg/mL | 762 | 206 | 955 | 334 |
| Heparan sulfate | 0.5 µg/mL | 837 | 248 | 995 | 407 |

### Example 16

ROTEM was used to analyze blood coagulation.

Blood was collected using a blood collection tube containing 3.2% sodium citrate manufactured by Terumo Corporation.

Blood coagulation was analyzed in the case of having added a rabbit brain-derived tissue thromboplastin reagent (TP; final concentration 16.6 µg/mL), calcium chloride (Ca; final concentration 12 mM), CTI (final concentration 20 µg/mL), and PKSI-527 (final concentration 40 µM), without and with a soluble thrombomodulin (rTM; final concentration: 100, 200, 300, 400, and 500 nM).

The CTs (clotting times) and CFT values (clot formation times) are shown in Table 6.

When the soluble thrombomodulin had been added in a concentration of 400 nM or higher, the clotting times were prolonged by 1.5 times or more even without heparan sulfate.

**[Table 6]**

| TP 16.6 µg/mL+Ca 12mM + CTI 20 µg/mL + PKSI-527 40µM + rTM 0-500 nM | | | | | | | | | | | |
|---|---|---|---|---|---|---|---|---|---|---|---|
| no addition of rTM | | rTM 100 nM | | rTM 200 nM | | rTM 300 nM | | rTM 400 nM | | rTM 500 n M | |
| CT (sec) | CFT (sec) | CT (sec) | CFT (sec) | CT (sec) | CFT (sec) | CT (sec) | CFT (sec) | CT (sec) | CFT (sec) | CT (sec) | CFT (sec) |
| 50 | 92 | 49 | 76 | 52 | 64 | 59 | 64 | 79 | 53 | 138 | 65 |

### Example 17

ROTEM was used to analyze blood coagulation.

Blood was collected using a blood collection tube containing 3.2% sodium citrate manufactured by Terumo Corporation.

Blood coagulation was analyzed in the case of having added a rabbit brain-derived tissue thromboplastin reagent (TP; final concentration 0.011 µg/mL), calcium chloride (Ca; final concentration 12 mM), heparan sulfate (final concentration 0, 0.2, 0.3, and 0.5 µg/mL), CTI (final concentration 20 µg/mL), and PKSI-527 (final concentration 40 µM) without and with a soluble thrombomodulin (rTM; final concentration 10 nM) further having been added.

The CTs (clotting times) and CFT values (clot formation times) are shown in Table 7.

In the case of having added the 0.011 µg/mL of thromboplastin and the heparan sulfate (0.2 µg/mL or more), the blood clotting times were 1 hour or longer when the soluble thrombomodulin had been added in a concentration of 10 nM, and none of the CT values were obtained.

**[Table 7]**

| TP 0.011 µg/mL + Ca 12mM+CTI 20 µg/mL + PKSI-527 40µM+Heparan sulfate 0∼0.5 µg/mL±rTM 10nM | | | | | |
|---|---|---|---|---|---|
| | | no addition of rTM | | rTM 10 nM | |
| | | CT (sec) | CFT (sec) | CT (sec) | CFT (sec) |
| Heparan sulfate | 0.0 µg/mL | 1139 | 263 | 1497 | 676 |
| Heparan sulfate | 0.2 µg/mL | 1408 | 578 | No CT value | No CFT value |
| Heparan sulfate | 0.3 µg/mL | 1630 | 430 | No CT value | No CFT value |
| Heparan sulfate | 0.5 µg/mL | 1178 | 386 | No CT value | No CFT value |

### Example 18

ROTEM was used to analyze blood coagulation.

Blood was collected using a blood collection tube containing 3.2% sodium citrate manufactured by Terumo Corporation.

Blood coagulation was analyzed in the case of having added a rabbit brain-derived tissue thromboplastin reagent (TP; final concentration 0.0166 µg/mL), calcium chloride (Ca; final concentration 12 mM), heparan sulfate (final concentration 0.3, 0.5, and 1 µg/mL), CTI (final concentration 20 µg/mL) and PKSI-527 (final concentration 40 µM) without and with soluble thrombomodulin (rTM; final concentration 10 nM) further having been added.

The CTs (clotting times) and CFT values (clot formation times) are shown in Table 8.

In the case of having added 0.0166 µg/mL of thromboplastin, the addition of the soluble thrombomodulin in a concentration of 10 nM did not prolong the CT values by 1.5 times or more. In the case of having added 1.0 µg/mL of heparan sulfate, the blood clotting times were 1 hour or longer and no CT values were obtained.

**[Table 8]**

| TP 0.0166 µg/mL + Ca 12mM + CTI 20 µg/mL+PKSI-527 40µM+Heparan sulfate 0.3∼1.0 µg/mL±rTM 10nM | | | | | |
|---|---|---|---|---|---|
| | | no addition of rTM | | rTM 10 nM | |
| | | CT (sec) | CFT (sec) | CT (sec) | CFT (sec) |
| Heparan sulfate | 0.3 µg/mL | 1035 | 256 | 904 | 292.0 |
| Heparan sulfate | 0.5 µg/mL | 1162 | 407 | 1064 | 742.0 |
| Heparan sulfate | 1.0 µg/mL | 1999 | No CT value | No CT value | No CFT value |

### Example 19

ROTEM was used to analyze blood coagulation.

Blood was collected using a blood collection tube containing 3.2% sodium citrate manufactured by Terumo Corporation.

Blood coagulation was analyzed in the case of having added a rabbit brain-derived tissue thromboplastin reagent (TP; final concentration 16.6 µg/mL), calcium chloride (Ca; final concentration 12 mM), an unfractionated heparin (MOCHIDA PHARMACEUTICAL CO., LTD.) (final concentration 0, 0.1, 0.2, and 0.3 U/mL), CTI (final concentration 20 µg/mL), and PKSI-527 (final concentration 40 µM) with and without a soluble thrombomodulin (rTM; final concentration 100 nM) further having been added.

The CTs (clotting times) and CFT values (clot formation times) are shown in Table 9.

The CT values for the unfractionated heparin (0 and 0.1 U/mL) at 16.6 µg/mL of thromboplastin were 46 and 53 seconds. Furthermore, no significant prolongation of CT values was observed with the addition of the 100 nM of soluble thrombomodulin.

When the thromboplastin with a concentration of 16.6 µg/mL and the unfractionated heparin (0 and 0.3 U/mL) had been added, on the other hand, the CT value was 69 seconds. Furthermore, the prolongation of CT values was markedly confirmed when 100 nM of soluble thrombomodulin had been added.

**[Table 9]**

| TP 16.6 µg/mL + Ca 12mM + CTI 20 µg/mL + PKSI-527 40µM + unfractionated heparin 0~0.3 U/mL± rTM 100nM | | | | | |
|---|---|---|---|---|---|
| | | no addition of rTM | | rTM 100 nM | |
| | | CT (sec) | CFT (sec) | CT (sec) | CFT (sec) |
| Unfractionated heparin | 0.0 U/mL | 46 | 123 | 46 | 90 |
| Unfractionated heparin | 0.1 U/mL | 53 | 98 | 66 | 93 |
| Unfractionated heparin | 0.2 U/mL | 73 | 82.0 | 89 | 102 |
| Unfractionated heparin | 0.3 U/mL | 69 | 86.0 | 182 | 161 |

### Example 20

ROTEM was used to analyze blood coagulation.

Blood was collected using a blood collection tube containing 3.2% sodium citrate manufactured by Terumo Corporation.

Blood coagulation was analyzed the case of having added a rabbit brain-derived tissue thromboplastin reagent (TP; final concentration 16.6 µg/mL), calcium chloride (Ca; final concentration 12 mM), fondaparinux sodium (trade name; ARIXTRA; final concentration 0, 0.5, 2, 3, 4, and 5 µg/mL), CTI (final concentration 20 µg/mL), and PKSI-527 (final concentration 40 µM), without and with a soluble thrombomodulin (rTM; final concentration100 nM) further having been added.

The CTs (clotting times) and CFT values (clot formation times) are shown in Table 10.

The CT values upon having added 16.6 µg/mL of thromboplastin and ARIXTRA (0, 0.5 µg/mL), were all shorter than 1 minute, and furthermore, and in the case of having added 100 nM of soluble thrombomodulin, no significant prolongation of CT values was observed.

The CT values upon having added 16.6 µg/mL of thromboplastin and ARIXTRA (2, 3, 4, and 5 µg/mL), on the other hand, ranged from 64 to 78 seconds, and furthermore, in the case of having added 100 nM of soluble thrombomodulin, the CT values were prolonged by 1.5 times or more in each case.

**[Table 10]**

| TP 16.6 µg/mL + Ca 12mM +CTI 20 µg/mL+ PKSI-527 40µM + ARIXTRA 0∼5 µg/mL± rTM 100nM | | | | | |
|---|---|---|---|---|---|
| | | no addition of rTM | | rTM 100 nM | |
| | | CT (sec) | CFT (sec) | CT (sec) | CFT (sec) |
| ARIXTRA | 0.0 µg/mL | 49 | 72 | 53 | 75 |
| ARIXTRA | 0.5 µg/mL | 52 | 86 | 68 | 69 |
| ARIXTRA | 2.0 µg/mL | 64 | 64.0 | 96 | 61 |
| ARIXTRA | 3.0 µg/mL | 72 | 78.0 | 112 | 68 |
| ARIXTRA | 4.0 µg/mL | 75 | 70.0 | 118 | 77 |
| ARIXTRA | 5.0 µg/mL | 78 | 77.0 | 114 | 105 |

### Example 21

ROTEM was used to analyze blood coagulation.

Blood was collected using a blood collection tube containing 3.2% sodium citrate manufactured by Terumo Corporation.

Blood coagulation was analyzed in the case of having added a rabbit brain-derived tissue thromboplastin reagent (TP; final concentration 0.64 µg/mL), calcium chloride (Ca; final concentration 12 mM), ARIXTRA (final concentration 0 and 0.5 µg/mL), CTI (final concentration 20 µg/mL), and PKSI-527 (final concentration 40 µM) without and with soluble thrombomodulin (rTM; final concentration 30 nM) further having been added.

The CTs (clotting times) and CFT values (clot formation times) are shown in Table 11.

The blood clotting times were significantly prolonged when the soluble thrombomodulin had been added in the presence of ARIXTRA.

**[Table 11]**

| TP 0.64 µg/mL + Ca 12mM + CTI 20 µg/mL + P KSI-527 40µM + A RIXTRA 0∼0.5 µg/ mL ± rTM 30nM | | | | | |
|---|---|---|---|---|---|
| | | no addition of rTM | | rTM 30 nM | |
| | | CT (sec) | CFT (sec) | CT (sec) | CFT (sec) |
| ARIXTRA | 0.0 µg/mL | 179 | 64 | 212 | 85 |
| ARIXTRA | 0.5 µg/mL | 294 | 74 | 581 | 794 |

### Example 22

ROTEM was used to analyze blood coagulation.

Blood was collected using a blood collection tube containing 3.2% sodium citrate manufactured by Terumo Corporation.

Blood coagulation was analyzed in the case of having added a rabbit brain-derived tissue thromboplastin reagent (TP; final concentration 0.055 µg/mL), calcium chloride (Ca; final concentration 12 mM), ARIXTRA (final concentration 0, 0.05, and 0.2 µg/mL), CTI (final concentration 20 µg/mL), and PKSI-517 (final concentration 40 µM) without and with soluble thrombomodulin (rTM; final concentration 20 nM) further having been added.

The CTs (clotting times) and CFT values (clot formation times) are shown in Table 12.

The CT value for 0.055 µg/mL of thromboplastin and no ARIXTRA was 575 seconds. Furthermore, no significant prolongation of CT values was observed in the case of having added 20 nM of soluble thrombomodulin.

With the addition of 0.055 µg/mL of thromboplastin and ARIXTRA (0.05 µg/mL), on the other hand, the CT values were both 619 seconds. Furthermore, when 20 nM of soluble thrombomodulin had been added, the CT values were prolonged by approximately 1.7 times.

Furthermore, when 0.055 µg/mL of thromboplastin and ARIXTRA (0.2 µg/mL) had been added, the CT values were both 799 seconds. Furthermore, when 20 nM of soluble thrombomodulin had been added, the blood clotting times were 1 hour or longer and no CT values were obtained.

**[Table 12]**

| TP 0.055 µg/mL + Ca 12mM + CTI 20 µg/mL + PKSI-527 40µM + ARIXTRA 0~0.2 µg/mL+rTM 20nM | | | | | |
|---|---|---|---|---|---|
| | | no addition of rTM | | rTM 20 nM | |
| | | CT (sec) | CFT (sec) | CT (sec) | CFT (sec) |
| ARIXTRA | 0.00 µg/mL | 575 | 126 | 715 | 165 |
| ARIXTRA | 0.05 µg/mL | 619 | 145 | 1074 | 403 |
| ARIXTRA | 0.20 µg/mL | 799 | 232 | No CT value | No CFT value |

### Example 23

ROTEM was used to analyze blood coagulation.

Blood was collected using a blood collection tube containing 3.2% sodium citrate manufactured by Terumo Corporation.

Blood coagulation was analyzed in the case of having added a rabbit brain-derived tissue thromboplastin reagent (TP; final concentration 16.6 µg/mL), calcium chloride (Ca; final concentration 12 mM), a low molecular weight heparin (trade name FRAGMIN; Pfizer Inc.) (final concentration 0, 0.2, and 1 U/mL), CTI (final concentration 20 µg/mL), and PKSI-527 (final concentration 40 µM ) without and with a soluble thrombomodulin (final concentration 100 nM) further having been added.

The CTs (clotting times) and CFT values (clot formation times) are shown in Table 13.

The CT values for 16.6 µg/mL of thromboplastin and FRAGMIN (0 and 0.2 U/mL) having added were 46 and 50 seconds. Furthermore, no significant prolongation of CT values was observed even with the addition of 100 nM of soluble thrombomodulin.

When 16.6 µg/mL of thromboplastin and FRAGMIN (1 U/mL) had been added, on the other hand, the CT value was 83 seconds. Furthermore, when 100 nM of soluble thrombomodulin had been added, the CT values were prolonged by approximately 1.5 times.

**[Table 13]**

| TP 16.6 µg/mL + Ca 12mM + CTI 20 µg/mL+ PKSI-527 40µM+ FRAGMIN 0∼1 U/mL±rTM 100nM | | | | | |
|---|---|---|---|---|---|
| | | no addition of rTM | | rTM 100 nM | |
| | | CT (sec) | CFT (sec) | CT (sec) | CFT (sec) |
| FRAGMIN | 0.0 U/mL | 46 | 123 | 46 | 90 |
| FRAGMIN | 0.2 U/mL | 50 | 97 | 62 | 89 |
| FRAGMIN | 1.0 U/mL | 83 | 78.0 | 130 | 321 |

### Example 24

ROTEM was used to analyze blood coagulation.

Blood was collected using a blood collection tube containing 3.2% sodium citrate manufactured by Terumo Corporation.

Blood coagulation was analyzed in the case of having added a rabbit brain-derived tissue thromboplastin reagent (TP; final concentration 0.64 µg/mL), calcium chloride (Ca; final concentration 12 mM), a low molecular weight heparin (FRAGMIN; Pfizer Inc.) (final concentration 0 and 0.2 U/mL), CTI (final concentration 20 µg/mL) and PKSI-527 (final concentration 40 µM) with and without a soluble thrombomodulin (rTM; final concentration 30 nM) further having added.

The CTs (clotting times) and CFT values (clot formation times) are shown in Table 14.

The CT values of the soluble thrombomodulin was significantly prolonged by the addition of FRAGMIN (0.2 U/mL).

**[Table 14]**

| TP 0.64 µg/mL + Ca 12mM + CTI 20 µg/mL + PKSI-527 40µM + FRAGMIN 0-0.2 U/mL± rTM 30nM | | | | | |
|---|---|---|---|---|---|
| | | no addition of rTM | | rTM 30 nM | |
| | | CT (sec) | CFT (sec) | CT (sec) | CFT (sec) |
| FRAGMIN | 0.0 U/mL | 157 | 68 | 186 | 65 |
| FRAGMIN | 0.2 U/mL | 248 | 67 | 782 | 1636 |

### Example 25

ROTEM was used to analyze blood coagulation.

Blood was collected using a blood collection tube containing 3.2% sodium citrate manufactured by Terumo Corporation.

Blood coagulation was analyzed in the case of having added a rabbit brain-derived tissue thromboplastin reagent (TP; final concentration 0.055 µg/mL), calcium chloride (Ca; final concentration 12 mM), a low molecular weight heparin (FRGMIN; Pfizer Inc.) (final concentration 0, 0.05, and 0.15 units/mL), CTI (final concentration 20 µg/mL), and PKSI-527 (final concentration 40 µM), with and without a soluble thrombomodulin (rTM; final concentration: 20 nM) further having been added.

The CTs (clotting times) and CFT values (clot formation times) are shown in Table 15.

The CT values for 0.055 µg/mL of thromboplastin and FRAGMIN (0, 0.05, and 0.15 units/mL), were 575, 614, and 886 seconds. Furthermore, when 20 nM of soluble thrombomodulin had been added, no significant prolongation of CT values was observed when FRAGMIN had not been added, however, the addition of 0.05 units/mL of RFRAGMIN prolonged blood coagulation by approximately 1.6 times after the addition of soluble thrombomodulin. When 0.15 units/mL of FRAGMIN had been added, the blood clotting times were 1 hour or longer with the addition of soluble thrombomodulin, and no CT values were obtained.

**[Table 15]**

| TP 0.055 µg/mL + Ca 12mM + CTI 20 µg/mL + PKSI-527 40µM + FRAGMIN 0∼0.15 U/mL±rTM 20nM | | | | | |
|---|---|---|---|---|---|
| | | no addition of rTM | | rTM 20 nM | |
| | | CT (sec) | CFT (sec) | CT (sec) | CFT (sec) |
| FRAGMIN | 0.00 U/mL | 575 | 126 | 715 | 165 |
| FRAGMIN | 0.05 U/mL | 614 | 104 | 980 | 225 |
| FRAGMIN | 0.15 U/mL | 886 | 239 | No CT value | No CFT value |

### Example 26

ROTEM was used to analyze blood coagulation.

Blood was collected using a blood collection tube containing 3.2% sodium citrate manufactured by Terumo Corporation.

Blood coagulation was analyzed in the case of having added 4% (final concentration 0.569 µg/mL) of Dade Innovin (recombinant tissue factor: Sysmex Corporation) having being diluted by 50 times after dialysis, calcium chloride (Ca; final concentration 12 mM), heparan sulfate (final concentration 0 or 2 µg/mL), CTI (final concentration 20 µg/mL), PKSI -527 (final concentration 40 µM) without and with a soluble thrombomodulin (rTM; final concentration 30 nM) further having been added, as well as with an APC inhibitory aptamer (HS02-44G; final concentration 1 µM) in addition to 30 nM of soluble thrombomodulin.

The CTs (clotting times) are shown in Table 16.

The anticoagulation ability of the soluble thrombomodulin was markedly enhanced by the addition of heparan sulfate. Moreover, the inhibition of activated protein C also shortened the clotting time. The shortening range may reflect the anticoagulation ability via protein C and protein S.

**[Table 16]**

| Dialyzed Dade Innovin 0.569 µg/mL + Ca 12mM + CTI 20 µg/mL + PKSI-527 40µM + heparan sulfate ± rTM 30nM± APC inhibitory aptamer 0~2 µg/mL 1 µM | | | |
|---|---|---|---|
| | rTM no addition | rTM 30nM | rTM 30nM + APC inhibitory aptamer 1 µM |
| | CT (sec) | CT (sec) | CT (sec) |
| heparan sulfate 0 µg/mL | 189 | 229 | 202 |
| heparan sulfate 2 µg/mL | 410 | 904 | 643 |

### Example 27

ROTEM was used to analyze blood coagulation.

Blood was collected using a blood collection tube containing 3.2% sodium citrate manufactured by Terumo Corporation.

Blood coagulation was analyzed in the case of having added 4% (final concentration 0.569 µg/mL) of 50-fold diluted Dade Innovin (recombinant tissue factor: Sysmex Corporation) after dialysis, calcium chloride (Ca; final concentration 12 mM), low molecular weight heparan (trade name FRAGMIN; Pfizer) (final concentration 0 or 0.2 U/mL), CTI (final concentration 20 µg/mL), and PKSI-527 (final concentration 40 µ M) with and without a soluble thrombomodulin (rTM; final concentration 30 nM) further having been added, as well as with an APC inhibitory aptamer (HS02-44G; final concentration 1 µM) in addition to 30 nM soluble thrombomodulin.

The CTs (clotting times) are shown in Table 17. The anticoagulation ability of the soluble thrombomodulin was markedly enhanced by the addition of FRAGMIN. Moreover, the inhibition of activated protein C shortened the clotting time. The shortening range may reflect the anticoagulation ability via protein C and protein S.

**[Table 17]**

| Dialyzed Dade Innovin 0.569 µg/mL + Ca 12mM + CTI 20 µg/mL + PKSI-527 40µM + FRAGMIN 0-0.2 U/mL ± rTM 30nM ± APC inhibitory aptamer 1µM | | | |
|---|---|---|---|
| | rTM no addition | rTM 30nM FRAGMIN | rTM 30nM + APC inhibitory aptamer 1 µM |
| | CT (sec) | CT(sec) | CT(sec) µM |
| FRAGMIN 0.0U/mL | 189 | 239 | 235 |
| FRAGMIN 0.2U/mL | 367 | 775 | 512 |

### Example 28

ROTEM was used to analyze coagulation.

Blood was collected using a blood collection tube containing 3.2% sodium citrate manufactured by Terumo Corporation.

Blood coagulation was analyzed in the case of having added 4% (final concentration 0.569 µg/mL) of 50-fold diluted Dade Innovin (recombinant tissue factor: Sysmex Corporation) after dialysis, calcium chloride (Ca; final concentration 12 mM), ARIXTRA (final concentration 0 or 0.5 µ/mL), CTI (final concentration 20 µg/mL), and PKSI-527 (final concentration 40 µM), without and with thrombomodulin (rTM; final concentration 30 nM) further having been added, as well as with an APC inhibitory aptamer (HS02-44G; final concentration 1 µM) in addition to soluble thrombomodulin 30 nM.

The CTs (clotting times) are shown in Table 18. The anticoagulation ability of the soluble thrombomodulin was markedly enhanced by the addition of ARIXTRA. Moreover, the inhibition of activated protein C shortened the clotting time. The shortening range may reflect the anticoagulation ability via Protein C and Protein S.

**[Table 18]**

| Dialyzed Dade Innovin 0.569µg/mL + Ca 12mM + CTI 20 µg/mL + PKSI-527 40µM + ARIXTRA 0-0.5 µg/mL ± rTM 30nM ± APC inhibitory aptamer 1µM | | | |
|---|---|---|---|
| | No addition of rTM | rTM 30 nM | rTM 30 nM + APC inhibitory aptamer 1µM |
| | CT (sec) | CT (sec) | CT (sec) |
| ARIXTRA 0.0µg/mL | 189 | 239 | 235 |
| ARIXTRA 0.5µg/mL | 466 | 996 | 474 |

### Example 29

### Measurement of PT values of rabbit brain-derived tissue thromboplastin and Dade Innovin

The PT values of the tissue factors used in Examples were measured by using a semiautomatic blood coagulation analyzer CA104 (Sysmex Corporation).

The rabbit brain-derived tissue thromboplastin (415 µg/mL) was diluted in Buffer (5 mM Hepes, 0.15 M NaCl (pH 7.4)) containing 100 µg/mL human albumin. Dade Innovin was dialyzed in distilled water (711.3 µg/mL) and then diluted by Buffer containing 100 µg/mL of human albumin (5 mM Hepes, 0.15 M NaCl (pH 7.4)).

50 µL of standard plasma was incubated at 37°C for 1 minute, then mixed with 50 µL of a calcium chloride solution (25 mM) and 50 µL of diluted rabbit brain-derived tissue thromboplastin or 50 µL of a Dade Innovin solution, and a clotting time (PT value) was measured. The standard plasma used was Dade Ci-Trol Level 1 (Sysmex Corporation), standard human plasma for blood coagulation tests (Sysmex Corporation), and Pooled Normal Plasma (George King Bio-Medical, Inc.).

The results are shown in Tables 19 and 20.

**[Table 19]**

| Plasma coagulation time (sec) | | | |
|---|---|---|---|
| Rabbit brain-derived tissue thromboplastin (415µg/mL) | Standard Plasma | | |
| | Dade ci-trol level 1 | Standard human plasma for blood coagulation tests | Pooled Normal Plasma |
| Undiluted solution | 20.1 | 19.7 | 20.7 |
| 2 fold dilution | 18.0 | 18.4 | 19.3 |
| 5 fold dilution | 19.0 | 19.7 | 20.4 |
| 10 fold dilution | 22.6 | 23.4 | 24.0 |
| 25 fold dilution | 29.2 | 30.1 | 36.4 |
| 50 fold dilution | 36.0 | 39.0 | 41.9 |
| 75 fold dilution | 43.6 | 48.0 | 53.5 |
| 100 fold dilution | 47.9 | 51.7 | 57.9 |
| 200 fold dilution | 61.8 | 69.3 | 73.8 |
| 300 fold dilution | 74.1 | 83.4 | 87.9 |
| 400 fold dilution | 83.0 | 93.6 | 98.1 |
| 500 fold dilution | 89.7 | 101.0 | 105.1 |

**[Table 20]**

| Plasma coagulation time (sec) | | | |
|---|---|---|---|
| Dialyzed Dade Innovin (711.3 µg/mL) | Standard Plasma | | |
| | Dade ci-trol level 1 | Standard human plasma for blood coagulation tests | Pooled Normal Plasma |
| Undiluted solution | 13.7 | 13.7 | 13.8 |
| 2 fold dilution | 14.6 | 16.8 | 15.9 |
| 5 fold dilution | 18.8 | 19.6 | 18.4 |
| 10 fold dilution | 23.8 | 24.4 | 23.6 |
| 25 fold dilution | 32.2 | 31.9 | 30.6 |
| 50 fold dilution | 39.4 | 40.8 | 35.9 |
| 75 fold dilution | 45.8 | 45.5 | 42.6 |
| 100 fold dilution | 49.4 | 51.1 | 46.0 |
| 200 fold dilution | 64 | 69.2 | 60.4 |
| 300 fold dilution | 71.7 | 77.6 | 64.9 |
| 400 fold dilution | 78.5 | 85.9 | 71.0 |
| 500 fold dilution | 80.9 | 86.3 | 72.2 |

The above results indicate that the addition of 4% of the tissue thromboplastin or the recombinant tissue factor, which has a prothrombin time of 19 to 105 seconds in standard plasma, to blood, is suitable as an extrinsic blood coagulation activator.

### Example 30

ROTEM was used to analyze blood coagulation.

Blood was collected using a blood collection tube containing 3.2% sodium citrate manufactured by Terumo Corporation.

The following reagents (1) to (4) below were added for a blood coagulation test.
(1) A rabbit brain-derived tissue thromboplastin reagent (TP; final concentration 0.664 µg/mL), calcium chloride (final concentration 12 mM) and heparan sulfate (final concentration 1 µg/mL)
(2) A rabbit brain-derived tissue thromboplastin reagent (TP; 0.664 µg/mL final concentration), calcium chloride (Ca; final concentration 12 mM), heparan sulfate (final concentration 1 µg/mL) and CTI (final concentration 20 µg/mL)
(3) A rabbit brain-derived tissue thromboplastin reagent (TP; final concentration 0.664 µg/mL), calcium chloride (Ca; final concentration 12 mM), heparan sulfate (final concentration 1 µg/mL) and PKSI-527 (final concentration 40 µM)
(4) A rabbit brain-derived tissue thromboplastin reagent (TP; final concentration 0.664 µg/mL), calcium chloride (Ca; final concentration 12 mM), heparan sulfate (final concentration 1 µg/mL), CTI (final concentration 20 µg/mL) and PKSI-527 (final concentration 40 µM).

Furthermore, to the reagents (1) to (4) were further added a soluble thrombomodulin (rTM; final concentration 25 nM) or a soluble thrombomodulin (rTM; final concentration 25 nM) and an APC inhibitory aptamer (HS02-44G; final concentration 1 µM) to then analyze blood coagulation.

The results are shown below and the clotting times (CTs) are shown in Table 21.

**[Table 21]**

| | | No addition of rTM | rTM 25nM | rTM 25nM + APC inhibitory aptamer 1 µM |
|---|---|---|---|---|
| | | CT (sec) | CT (sec) | CT (sec) |
| (1) | TP 0.664µg/mL + Heparan sulfate 1µg/mL + Ca 12mM | 220 | 533 | 260 |
| (2) | TP 0.664µg/mL + Heparan sulfate 1µg/mL + Ca 12mM + CTI 20µg/mL | 214 | 448 | 287 |
| (3) | TP 0.664µg/mL + Heparan sulfate 1µg/mL + Ca 12mM + PKSI-527 40µM | 220 | 444 | 291 |
| (4) | TP 0.664µg/mL + Heparan sulfate 1µg/mL + Ca 12mM + CTI 20µg/mL + PKSI-527 40µM | 226 | 440 | 319 |

The addition of heparan sulfate markedly prolonged the blood clotting time with the soluble thrombomodulin under all conditions, and the addition of the APC inhibitor further shortened the clotting time.

### Example 31

ROTEM was used to analyze blood coagulation.

Blood was collected using a blood collection tube containing 3.2% sodium citrate manufactured by Terumo Corporation.

The following reagents (1) to (4) below were added for a blood coagulation test.
(1) A rabbit brain-derived tissue thromboplastin reagent (TP; final concentration 0.664 µg/mL), calcium chloride (Ca; final concentration 12 mM) and heparan sulfate (final concentration 1 µg/mL)
(2) A rabbit brain-derived tissue thromboplastin reagent (TP; 0.664 µg/mL final concentration), calcium chloride (Ca; final concentration 12 mM), heparan sulfate (final concentration 1 µg/mL), and CTI (final concentration 20 µg/mL)
(3) A rabbit brain-derived tissue thromboplastin reagent (TP; 0.664 µg/mL final concentration), calcium chloride (Ca; final concentration 12 mM), heparan sulfate (final concentration 1 µg/mL), and PKSI-527 (final concentration 40 µM)
(4) A rabbit brain-derived tissue thromboplastin reagent (TP; 0.664 µg/mL final concentration), calcium chloride (Ca; final concentration 12 mM), heparan sulfate (final concentration 1 µg/mL), CTI (final concentration 20 µg/mL) and PKSI-527 (final concentration 40 µM)

Furthermore, to the reagents (1) to (4) were further added a soluble thrombomodulin (rTM; final concentration 30 nM) or a soluble thrombomodulin (rTM; final concentration 30 nM) and an APC inhibitory aptamer (HS02-44G; final concentration 1 µM) to then analyze blood coagulation.

The results are shown below, and the clotting time (CT) is shown in Table 22.

**[Table 22]**

| | | No addition of rTM | rTM 30nM | rTM 30nM + APC inhibitory aptamer 1 µM |
|---|---|---|---|---|
| | | CT (sec) | CT (sec) | CT (sec) |
| (1) | TP 0.664µg/mL + Heparan sulfate 1µg/mL + Ca 12mM | 220 | 766 | 301 |
| (2) | TP 0.664µg/mL + Heparan sulfate 1µg/mL + Ca 12mM + CTI 20µg/mL | 214 | 613 | 286 |
| (3) | TP 0.664µg/mL + Heparan sulfate 1µg/mL + Ca 12mM + PKSI-527 40µM | 220 | 517 | 297 |
| (4) | TP 0.664µg/mL + Heparan sulfate 1µg/mL + Ca 12mM + CTI 20µg/mL + PKSI-527 40µM | 226 | 557 | 307 |

The addition of heparan sulfate markedly prolonged the blood clotting time with the soluble thrombomodulin under all conditions, and the addition of the APC inhibitor further shortened the clotting time.

### Example 32

ROTEM was used to analyze blood coagulation.

Blood was collected using a blood collection tube containing 3.2% sodium citrate manufactured by Terumo Corporation.

The following reagents (1) to (4) were added for a blood coagulation test.
(1) A rabbit brain-derived tissue thromboplastin reagent (TP; final concentration 0.664 µg/mL), calcium chloride (Ca; final concentration 12 mM), CTI (final concentration 20 µg/mL), PKSI-527 (final concentration 40 µM) and dextran sulfate (Meito Sangyo Co., Ltd., dextran sulfate sodium sulfur 18 (average molecular weight 5,000, final concentration 60 µg/mL)
(2) A rabbit brain-derived tissue thromboplastin reagent (TP; final concentration 0.664 µg/mL), calcium chloride (Ca; final concentration 12 mM), CTI (final concentration 20 µg/mL), PKSI-527 (final concentration 40 µM), and dextran sulfate (Meito Sangyo Co., Ltd., dextran sulfate sodium sulfur 18, average molecular weight 5,000, final concentration 60 µg/mL) and a soluble thrombomodulin (rTM; final concentration 40 nM)
(3) A rabbit brain-derived tissue thromboplastin reagent (TP; 0.664 µg/mL final concentration), calcium chloride (Ca; final concentration 12 mM), CTI (final concentration 20 µg/mL), PKSI-527 (final concentration 40 µM), dextran sulfate (Meito Sangyo Co., Ltd., dextran sulfate sodium sulfur 18, average molecular weight 5,000, final concentration 60 µg/mL), a soluble thrombomodulin (rTM; final concentration 40 nM) and an APC inhibitory aptamer (HS02-44G; final concentration 1 µM)
(4) A rabbit brain-derived tissue thromboplastin reagent (TP; 0.664 µg/mL final concentration), calcium chloride (Ca; final concentration 12 mM), CTI (final concentration 20 µg/mL), PKSI-527 (final concentration 40 µM), dextran sulfate (Meito Sangyo Co., Ltd., dextran sulfate sodium sulfur 18, average molecular weight 5,000, final concentration 60 µg/mL), and an unfractionated heparin (final concentration 0.5 U/mL)
(5) A rabbit brain-derived tissue thromboplastin reagent (TP; final concentration 0.664 µg/mL), calcium chloride (Ca; final concentration 12 mM), CTI (final concentration 20 µg/mL), PKSI-527 (final concentration 40 µM), dextran sulfate (Meito Sangyo Co., Ltd., dextran sulfate sodium sulfur 18, average molecular weight 5,000, final concentration 60 µg/mL), and a soluble thrombomodulin (rTM; final concentration 40 nM) and an unfractionated heparin (final concentration 0.5 U/mL)
(6) A rabbit brain-derived tissue thromboplastin reagent (TP; final concentration 0.664 µg/mL), calcium chloride (Ca; final concentration 12 mM), CTI (final concentration 20 µg/mL), PKSI-527 (final concentration 40 µM), dextran sulfate (Meito Sangyo Co., Ltd., dextran sulfate sodium sulfur 18, average molecular weight 5,000, final concentration 60 µg/mL), a soluble thrombomodulin (rTM; final concentration 40 nM), an APC inhibitory aptamer (HS02-44G; final concentration 1 µM) and an unfractionated heparin (final concentration 0.5 U/mL)
(7) A rabbit brain-derived tissue thromboplastin reagent (TP; 0.664 µg/mL final concentration), calcium chloride (Ca; final concentration 12 mM), CTI (final concentration 20 µg/mL), PKSI-527 (final concentration 40 µM), dextran sulfate (Meito Sangyo Co., Ltd., dextran sulfate sodium sulfur 18, average molecular weight 5,000, final concentration 60 µg/mL), an unfractionated heparin (final concentration 0.5 units/mL) and heparinase I (IBEX Pharmaceuticals Inc., final concentration 1000 U/mL)
(8) A rabbit brain-derived tissue thromboplastin reagent (TP; 0.664 µg/mL final concentration), calcium chloride (Ca; final concentration 12 mM), CTI (final concentration 20 µg/mL), PKSI-527 (final concentration 40 µM), dextran sulfate (Meito Sangyo Co., Ltd., dextran sulfate sodium sulfur 18, average molecular weight 5,000, final concentration 60 µg/mL), a soluble thrombomodulin (rTM; final concentration 40 nM), an unfractionated heparin (final concentration 0.5 U/mL) and heparinase I (IBEX Pharmaceuticals Inc., final concentration 1000 U/mL)
(9) A rabbit brain-derived tissue thromboplastin reagent (TP; 0.664 µg/mL final concentration), calcium chloride (Ca; final concentration 12 mM), CTI (final concentration 20 µg/mL), PKSI-527 (final concentration 40 µM), dextran sulfate (Meito Sangyo Co., Ltd., dextran sulfate sodium sulfur 18, average molecular weight 5,000, final concentration 60 µg/mL), a soluble thrombomodulin (rTM, final concentration 40 nM), an APC inhibitory aptamer (HS02-44G; final concentration 1 µM), an unfractionated heparin (final concentration 0.5 U/mL), and heparinase I (IBEX Pharmaceuticals Inc., final concentration 1000 U/mL)

The results are shown below, and the clotting times (CTs) are shown in Table 23.

**[Table 23]**

| | rTM no addition | | rTM 40nM | | rTM 40nM + APC inhibitory aptamer 1 µM | |
|---|---|---|---|---|---|---|
| | | CT (sec) | | CT (sec) | | CT (sec) |
| TP 0.664µg/mL + Dextran sulfate sodium sulfur 18 1µg/mL + Ca 12mM + CTI 20µg/mL + PKSI-527 40µM | (1) | 241 | (2) | 385 | (3) | 250 |
| TP 0.664µg/mL + Dextran sulfate sodium sulfur 18 1µg/mL + Ca 12mM + CTI 20µg/mL + PKSI-527 40µM + Unfractionated heparin 0.5 unit/mL | (4) | 426 | (5) | No CT value | (6) | 771 |
| TP 0.664µg/mL + Dextran sulfate sodium sulfur 18 1µg/mL + Ca 12mM + CTI 20µg/mL + PKSI-527 40µM + Unfractionated heparin 0.5 unit/mL + Heparinase I 1000 unit/mL | (7) | 239 | (8) | 380 | (9) | 255 |

The addition of dextran sulfate as the heparin-like substance provides the favorable prolongation effect of TM. Furthermore, the addition of unfractionated heparin and heparinase also made it possible to evaluate the prolongation effect of soluble thrombomodulin. This is considered to enable the evaluation of anticoagulation ability via PS/PC by using dextran sulfate as the heparin-like substance, when evaluating blood of patients to which heparin is administered.

## Claims

1. A method of analyzing blood coagulation in vitro, comprising analyzing blood coagulation ability using a blood sample to which an extrinsic blood coagulation activator, thrombomodulin and a heparin-like substance are added.

2. The method according to claim 1, wherein the heparin-like substance is at least one selected from the group consisting of an unfractionated heparin, a low molecular weight heparin, pentasaccharide, and sulfated polysaccharide.

3. The method according to claim 1 or 2, wherein the heparin-like substance is heparan sulfate and is added at a final concentration of 0.4 to 15 µ/mL.

4. The method according to claim 1, wherein the extrinsic blood coagulation activator is a tissue factor or tissue thromboplastin.

5. The method according to any one of claims 1 to 4, further comprising adding a contact factor inhibitor.

6. The method according to any one of claims 1 to 5, wherein the tissue factor or tissue thromboplastin and the heparin-like substance are added in a concentration such that a blood clotting time is 60 to 700 seconds, and thrombomodulin is added thereto.

7. The method according to any one of claims 1 to 6, wherein the thrombomodulin is soluble thrombomodulin.

8. The method according to any one of claims 1 to 7, wherein a final concentration of thrombomodulin is from 9 nM to 200 nM.

9. The method according to any one of claims 1 to 8, wherein said blood coagulation ability analysis is performed by further adding a protein C inhibitor, and functions of protein C and protein S are evaluated by comparing the analysis result with that obtained when the protein C inhibitor is not added.

10. The method according to any one of claims 1 to 9, wherein
the blood sample is subjected to anticoagulation treatment with citric acid, and
blood coagulation is evaluated by adding calcium, the extrinsic blood coagulation activator, the thrombomodulin, and the heparin-like substance.

11. The method according to any one of claims 1 to 10, wherein a contact factor inhibitor is further added to the blood sample.

12. The method according to claim 11, wherein the contact factor inhibitor is either an FXII inhibitor or a kallikrein inhibitor, or both thereof.

13. The method according to any one of claims 1 to 12, wherein functions of protein C and protein S are evaluated by comparing the analysis result with that obtained when the thrombomodulin is not added.

14. Ablood coagulation analysis reagent comprising an extrinsic blood coagulation activator, thrombomodulin and a heparin-like substance.

15. The blood coagulation analysis reagent according to claim 14, further comprising a contact factor inhibitor.
